(19) Europäisches Patentamt
European Patent Office
Office européen des brevets

(11) **EP 2 378 146 A2**

(12) **EUROPÄISCHE PATENTANMELDUNG**

(43) Veröffentlichungstag:
**19.10.2011 Patentblatt 2011/42**

(51) Int Cl.:
*F16C 29/04* (2006.01)    *G01N 33/28* (2006.01)
*G01M 13/04* (2006.01)

(21) Anmeldenummer: **11161090.3**

(22) Anmeldetag: **05.04.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **16.04.2010 DE 102010015207**

(71) Anmelder: **Schaeffler Technologies GmbH & Co. KG**
**91074 Herzogenaurach (DE)**

(72) Erfinder:
• **Glück, Stefan**
**97424, Schweinfurt (DE)**
• **Benkert, Frank**
**97534, Waigolshausen (DE)**

(54) **Verfahren zur Überwachung einer Linearführung**

(57) Die Erfindung betrifft ein Verfahren zur Überwachung einer Linearführung mit einem auf einem geschmierten Wälzkontakt entlang einer Schiene verlagerbaren Wagen mit einer Sensoreinrichtung zur Erfassung eines Zustands eines Schmierstoffs zur Schmierung des Wälzkontakts. Um eine schnelle Ermittlung des Zustands des Schmiermittels mit einfachen Mitteln zu ermöglichen, wird mittels der Sensoreinrichtung ein Schwingungszeitsignal erfasst, aus dem nach einer Hochpassfilterung (4) mittels einer Effektivwertbestimmung (5) ein mit zunehmender Degradierung des Schmierstoffs ansteigender Kennwert (4) ermittelt wird, wobei bei einem Überschreiten des Kennwerts über einen vorgegebenen Schwellwert Maßnahmen zu einer Verbesserung der Schmiereigenschaften eingeleitet werden.

EP 2 378 146 A2

## Beschreibung

### Gebiet der Erfindung

Die Erfindung betrifft ein Verfahren zur Überwachung einer Linearführung mit einem auf einem geschmierten Wälzkontakt entlang einer Schiene verlagerbaren Wagen mit einer Sensoreinrichtung zur Erfassung eines Zustands eines Schmierstoffs zur Schmierung des Wälzkontakts.

### Hintergrund der Erfindung

Linearführungen dienen der Führung eines Wagens entlang einer Profilschiene. Zur Ausbildung des Führungskontakts zwischen Profilschiene und Wagen werden Gleit- und Wälzkontakte eingesetzt. Besonders günstig sind infolge der Wandlung einer Gleit- in eine Rollreibung Wälzkontakte. Dabei werden Wälzkörper entweder stationär oder umlaufend zwischen der Profilschiene und dem Wagen angeordnet. Zur weiteren Herabsetzung des Reibwiderstands und zur Verringerung von Verschleiß werden die Wälzkörper mit einem Schmierstoff, beispielsweise Öl- oder Fett versorgt, der zwischen Wälzkörpern und Laufbahnen dieser einen dünnen Schmierfilm bildet. Zur Bevorratung von Schmiermittel kann beispielsweise am Wagen ein Schmiermittelreservoir vorgesehen sein, aus dem gemäß entsprechender Wartungsvorschriften manuell oder automatisiert, beispielsweise nach vorgegebenen Betriebsintervallen Schmiermittel auf den Wälzkontakt zwischen Wälzkörper und Laufbahnen dosiert wird.

Der Schmierstoff hat bei ausreichender Ausbildung eines Schmierfilms am Wälzkontakt einen wesentlichen Einfluss auf die nominelle Lebensdauer der Linearführung.

Um eine ausreichende Ausbildung eines Schmierfilms in einer Linearführung überwachen zu können, wird in der DE 10 2006 017 203 A1 vorgeschlagen, eine Sensoreinrichtung zur Erfassung einzusetzen, die ein direktes oder indirektes Maß für das Vorhandensein eines ausreichenden Schmierfilms zwischen den Wälzkörpern und einer Lauffläche dieser erfasst. Hierbei wird als physikalische Messgröße mittels eines Strahlungsempfängers eine Strahlungsantwort erfasst, die einer Einstrahlung auf den relevanten Schmierbereich mittels einer Strahlungsquelle folgt.

### Aufgabe der Erfindung

Aufgabe der Erfindung ist die vorteilhafte Weiterbildung eines Verfahrens zur Überwachung eines Schmierfilms am Wälzkontakt zwischen den Wälzkörpern und deren Laufflächen einer Linearführung insbesondere vor dem Hintergrund einer einfachen und kostengünstigen Umsetzung des Verfahrens mittels einfacher Komponenten.

### Beschreibung der Erfindung

Die Aufgabe wird durch ein Verfahren zur Überwachung einer Linearführung mit einem auf einem geschmierten Wälzkontakt entlang einer Schiene verlagerbaren Wagen mit einer Sensoreinrichtung zur Erfassung eines Zustands eines Schmierstoffs zur Schmierung des Wälzkontakts gelöst, wobei mittels der Sensoreinrichtung ein Schwingungszeitsignal erfasst wird, aus dem Schwingungszeitsignal nach einer Hochpassfilterung mittels einer Effektivwertbestimmung ein mit zunehmender Degradierung des Schmierstoffs ansteigender Kennwert ermittelt wird und bei einem Überschreiten des Kennwerts über einen vorgegebenen Schwellwert Maßnahmen zu einer Verbesserung der Schmiereigenschaften eingeleitet werden.

Mittels des vorgeschlagenen Verfahrens können alle Formen von Linearführungen mit geschmiertem Wälzkontakt, insbesondere als Kugelumlaufeinheiten ausgebildete Linearführungen auf einen ausreichenden Schmierfilm überwacht werden. Zur konstruktiven Ausführung von Linearführungen wird auf bekannte Ausführungsformen, wie sie beispielweise in der DE 10 2006 017 203 A1 offenbart sind, verwiesen.

Als Maßnahmen zur Verbesserung der Schmiereigenschaften können beispielsweise eine automatische Dosierung von Schmierstoff und/oder ein Alarmsignal sein. Beispielsweise kann bei einer Überschreitung des Schwellwerts eine automatisierte Dosierung von Schmierstoff aus einem Vorratsbehälter, der beispielsweise an dem Wagen oder an der Profilschiene angeordnet sein kann, erfolgen, wobei eine Dosiermenge abhängig vom Maß des überschrittenen Schwellwerts eingestellt werden kann. Alternativ oder zusätzlich kann das Alarmsignal ausgegeben werden. In weiteren Ausführungsbeispielen kann bei sehr schlecht ausgebildeter oder fehlender Schmierschicht die Linearführung stillgelegt werden, indem beispielsweise ein Antrieb wie Elektromotor oder dergleichen dieser, beispielsweise des auf der Profilschiene linear verlagerbaren Wagens, in eine Steuerroutine der Ermittlung, Erfassung und Auswertung des Signalzeitverlaufs einbezogen wird.

Bei der Hochpassfilterung handelt es sich bevorzugt um eine einfache Filterung, beispielsweise mittels eines analog oder digital darstellbaren Bessel-Filters, das bevorzugt höherer Ordnung, beispielsweise fünfter Ordnung sein kann, so dass der Aufbau der Sensoreinrichtung einfach und mittels eines einfachen Mikroprozessors erfolgen kann. Zur Begrenzung des zu erfassenden Frequenzbereichs kann vor der Hochpassfilterung eine Tiefpassfilterung durchgeführt werden, so dass niederfrequente Schwingungen, beispielsweise Sensorresonanzen und parasitäre Schwingungen ausgeblendet werden. Es hat sich als vorteilhaft erwiesen, wenn mittels der Tiefpassfilterung eine Bandbreite des Schwingungszeitsignals auf 16 kHz, vorzugsweise 14 kHz begrenzt wird. Erfolgt danach eine Hochpassfilterung für Frequenzen größer 8 kHz, vorzugsweise größer 11 kHz, kann ein vergleichsweise schmales Frequenzband erfasst werden, das weit unter den Frequenzen für Körperschallmessungen liegt. Es hat sich dabei überraschend herausgestellt, dass bei Verwendung des vorgeschlagenen Verfahrens in diesem Frequenzbereich eine Korrelation zwischen einem von den Effektivwerten eines Schwingungszeitsignals abgeleiteten Kennwerts und dem Schmierzustand hergestellt werden kann. Auf diese Weise kann mit einfachen, digital und analog darstellbaren Mitteln ein kostengünstiges Verfahren auf Basis einer entsprechenden Vorrichtung mit einer Sensoreinrichtung vorgeschlagen werden, bei dem einfache Sensoren, die bis zu Frequenzen von 16 kHz, vorzugsweise 14 kHz ausreichend zuverlässig arbeiten, verwendet werden können. Derartige Sensoren können beispielsweise piezoelektrische Sensoren, mittels Mikrosystemtechnik hergestellte Sensoren oder dergleichen sein, die zum Einen preisgünstig und zum Anderen miniaturisierbar herstellbar sind. Dementsprechend können diese Sensoren ohne hohen Bauraumbedarf vorteilhafterweise an dem Wagen der Linearführung angeordnet werden.

Es hat sich weiterhin als vorteilhaft erwiesen, wenn das Schwingungszeitsignal unter definierten Messbedingungen zur Vergleichbarkeit des erfassten Schwingungszeitsignals mit unter Referenzbedingungen erfassten Schwingungszeitsignalen erfasst wird. Auf diese Weise können von vorneherein in diesem Frequenzbereich auftretende Störeinflüsse, beispielsweise für die Linearführung typische Artefakte und dergleichen eliminiert werden. Derartige Störeinflüsse können beispielsweise in dem Schwellwert berücksichtigt werden. Dieser kann im einfachsten Fall eine über den Frequenzbereich gemittelte Größe darstellen oder aus einem Satz frequenzabhängiger Größen gebildet sein. Nach dem erfinderischen Gedanken werden definierte Messbedingungen erzielt, indem die Schwingungszeitsignale während zumindest einer Messfahrt des Wagens bei bekannter Geschwindigkeit und bekannter Last erfasst werden. Dabei ist eine konstante Einhaltung der Geschwindigkeit über den Weg des Wagens entlang der Profilschiene bevorzugt vorgesehen, sich ändernde Geschwindigkeiten können jedoch ebenfalls zur Erzielung spezieller Messeffekte angewendet werden, wobei das Geschwindigkeitsprofil reproduzierbar vorgegeben und die daraus ermittelten Effektivwerte Kennwerte liefern, die mit Schwellwerten verglichen werden, die unter Zugrundelegung derselben Geschwindigkeitsprofile festgelegt werden. Eine Messfahrt des Wagens kann dabei innerhalb einer kurzen Zeitspanne, beispielsweise zwischen zwei Arbeitsgängen der in einer Werkzeugmaschine integrierten Linearführung, durchgeführt werden, die beispielsweise kleiner drei Sekunden beträgt und vorzugsweise im Bereich einer Sekunde liegt.

Die Anordnung der Sensoreinrichtung beziehungsweise des Sensors der Sensoreinrichtung, beispielsweise ein piezoelektrischer Sensor, erfolgt bevorzugterweise in dem gegen die Profilschiene verlagerbaren Wagen. Bezogen auf dessen Bewegungsebene bezüglich seiner transversalen Bewegung wird der Sensor mit seiner Messachse oder geometrischen Achse bevorzugt normal, das heißt im Wesentlichen senkrecht zu dieser angeordnet. Alternativ kann der Sensor bezüglich dieser Achsen quer oder parallel zur transversalen Bewegung außerhalb oder in der Bewegungsebene untergebracht wie befestigt sein.

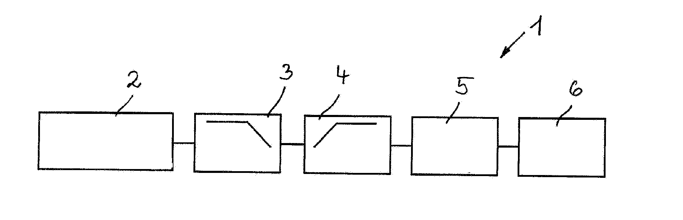

## Kurze Beschreibung der Zeichnung

Die Erfindung wird anhand des in der einzigen Figur dargestellten Ausführungsbeispiels näher erläutert. Diese zeigt ein Blockschaltbild eines Verfahrens zur Ermittlung eines eine Degradierung des Schmierstoffs einer Linearführung anzeigenden Kennwerts.

## Ausführliche Beschreibung der Zeichnungen

Die einzige Figur zeigt das Blockschaltbild 1 zur Durchführung eines Verfahrens zur Überprüfung des Zustands eines Schmierfilms am Wälzkontakt einer Linearführung zwischen Wälzkörpern und den zugehörigen Laufbahnen dieser. In Block 2 werden über einen Zeitraum einer Messfahrt des Wagens die Schwingungszeitdaten der Sensoreinrichtung, beispielsweise die Messsignale eines piezoelektrischen Sensors, der senkrecht zur Bewegungsebene des Wagen angeordnet ist, in eine Datenerfassungseinrichtung, beispielsweise einen in einem Mikroprozessor vorhandenen oder diesem zugeordneten flüchtigen oder nicht flüchtigen Speicher eingelesen. Die vorzugsweise aus dem Sensor als analoge, den auftretenden Schwingungen während der Messfahrt zugeordnete Daten werden dabei zuvor beispielsweise mittels eines A/D-Wandlers digitalisiert. In Block 3 werden die digitalisierten Daten mittels einer Filtereinheit tiefpassgefiltert, indem beispielsweise Frequenzen über 14 kHz abgeschnitten werden. Eine entsprechende digital arbeitende Filtereinheit in Form eines Tiefpassfilters kann beispielsweise ein Bessel-Filter fünfter Ordnung, ein Butterworth-Filter oder dergleichen sein. Alternativ können die analogen Daten mittels eines diskret aus Hardwarebausteinen aufgebauten Tiefpassfilters gefiltert werden. Hierbei können die Daten analog weiterverarbeitet oder an dieser Stelle digitalisiert werden. In Block 4 werden die bis dahin verarbeiteten Daten einem Hochpassfilter unterworfen, der bei Vorliegen der Daten in analoger Form wiederum aus Hardwarekomponenten oder bei digital vorliegenden Daten aus in einem Mikroprozessor abgearbeiteten algorithmischen Rechenschritten aufgebaut sein kann. Der Hochpassfilter lässt aus der Tiefpassfilterung verbleibende Schwingungssignale vorzugsweise größer 11 kHz passieren, so dass ein Frequenzbereich wie Frequenzband zwischen 11 und 14 kHz in Block 5 der Effektivwertbestimmung zugeführt wird und aus den Schwingungszeitsignalen $X_i$ mittels des Zusammenhangs

$$x_{eff} = \sqrt{\frac{1}{n}\sum x_i^2}$$

der Effektivwert X_{eff} ermittelt wird. In Block 6 wird der Effektivwert X_{eff} in einen Kennwert umgeformt, der beispielsweise auf eine Referenzgröße normiert oder in anderer Weise bearbeitet wird und einer Größe für das verwendete Frequenzband entspricht. Beobachtungen haben gezeigt, dass der Kennwert mit zunehmender Degradation des Schmierstoffs ansteigt, so dass in Block 5 ein Vergleich mit einem Schwellwert erfolgen kann, der eine noch ausreichende Qualität des Schmierstoffs zur Bildung des Schmierfilms markiert. Übersteigt der Kennwert den Schwellwert werden in derselben Routine oder in einer weiteren Routine Maßnahmen zur Verbesserung beziehungsweise Aufrechterhaltung des Schmierfilms getroffen, beispielsweise eine in der Linearführung vorhandene Dosiervorrichtung zur Dosierung von Schmierstoff aktiviert, ein Alarmsignal für Bedienpersonal ausgegeben und/oder andere Schritte eingeleitet.

**Patentansprüche**

1.  Verfahren zur Überwachung einer Linearführung mit einem auf einem geschmierten Wälzkontakt entlang einer Schiene verlagerbaren Wagen mit einer Sensoreinrichtung zur Erfassung eines Zustands eines Schmierstoffs zur Schmierung des Wälzkontakts, **dadurch gekennzeichnet, dass** mittels der Sensoreinrichtung ein Schwingungszeitsignal erfasst wird, aus dem Schwingungszeitsignal nach einer Hochpassfilterung mittels einer Effektivwertbestimmung ein mit zunehmender Degradierung des Schmierstoffs ansteigender Kennwert ermittelt wird und bei einem Überschreiten des Kennwerts über einen vorgegebenen Schwellwert Maßnahmen zu einer Verbesserung der Schmiereigenschaften eingeleitet werden.

2.  Verfahren nach Anspruch 1, **dadurch gekennzeichnet, dass** die Maßnahmen zur Verbesserung der Schmiereigenschaften eine automatische Dosierung von Schmierstoff und/oder ein Alarmsignal sind.

3.  Verfahren nach Anspruch 1 oder 2, **dadurch gekennzeichnet, dass** vor der Hochpassfilterung eine Tiefpassfilterung durchgeführt wird.

4.  Verfahren nach Anspruch 3, **dadurch gekennzeichnet, dass** mittels der Tiefpassfilterung eine Bandbreite des Schwingungszeitsignals auf 16 kHz, vorzugsweise 14 kHz begrenzt wird.

5.  Verfahren nach einem der Ansprüche 1 bis 4, **dadurch gekennzeichnet, dass** die Hochpassfilterung für Frequenzen größer 8 kHz, vorzugsweise größer 11 kHz durchlässig ist.

6.  Verfahren nach einem der Ansprüche 1 bis 5, **dadurch gekennzeichnet, dass** die Schwingungszeitsignale während zumindest einer Messfahrt des Wagens bei bekannter Geschwindigkeit und bekannter Last erfasst werden.

7.  Verfahren nach Anspruch 6, **dadurch gekennzeichnet, dass** die Messfahrt innerhalb 3 Sekunden, vorzugsweise im Bereich einer Sekunde durchgeführt wird.

8.  Verfahren nach einem der Ansprüche 1 bis 7, **dadurch gekennzeichnet, dass** die Sensoreinrichtung bezüglich einer Bewegungsebene des Wagens normal auf dem Wagen befestigt ist.

9.  Verfahren nach einem der Ansprüche 1 bis 7, **dadurch gekennzeichnet, dass** die Sensoreinrichtung bezüglich einer Bewegungsebene parallel und quer zur Bewegungsrichtung angeordnet ist.

10. Verfahren nach einem der Ansprüche 1 bis 9, **dadurch gekennzeichnet, dass** die Sensoreinrichtung einen piezoelektrischen Sensor oder einen mittels Mikrosystemtechnik hergestellten Sensor aufweist.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006017203 A1